(19) Europäisches Patentamt
European Patent Office
Office européen des brevets

(11) Veröffentlichungsnummer: **0 416 361 A1**

(12) **EUROPÄISCHE PATENTANMELDUNG**

(21) Anmeldenummer: **90115966.5**

(22) Anmeldetag: **21.08.90**

(51) Int. Cl.5: **C07D 207/452**, C07D 207/02, C07D 233/96, C07D 263/44, A01N 43/36, A01N 43/76, A01N 43/50

(30) Priorität: **02.09.89 DE 3929170**

(43) Veröffentlichungstag der Anmeldung: **13.03.91 Patentblatt 91/11**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Ooms, Pieter, Dr.**
**Doerperhofstrasse 16**
**W-4150 Krefeld 1(DE)**
Erfinder: **Lürssen, Klaus, Dr.**
**August-Kierspel-Strasse 145**
**W-5060 Bergisch-Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**
Erfinder: **Krauskopf, Birgit, Dr.**
**Kicke 19**
**W-5060 Bergisch-Gladbach 1(DE)**

(54) **N-(5-Alkylthiophenyl)-Stickstoffheterocyclen.**

(57) Neue N-(5-Alkylthiophenyl)-Stickstoffheterocyclen der allgemeinen Formel (I)

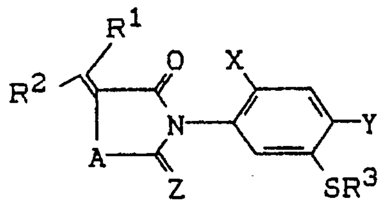

(I)

in welcher
$R^1$ und $R^2$ entweder unabhängig voneinander jeweils für Wasserstoff oder Alkyl stehen oder gemeinsam für einen zweifach verknüpften Alkandiylrest stehen,
$R^3$ für Wasserstoff, für jeweils gegebenenfalls substituiertes Alkyl, Alkenyl, Alkinyl, Cycloalkyl, Arylalkyl oder Heteroarylalkyl steht,
X für Wasserstoff oder Halogen steht,
Y für Halogen oder Alkyl steht,
Z für Sauerstoff oder Schwefel steht,
A für Sauerstoff, für eine CH-$R^4$-Gruppe, für eine

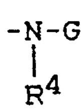-Gruppe oder für eine 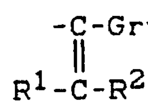-Gruppe steht,

EP 0 416 361 A1

wobei
$R^4$ für Wasserstoff, für jeweils gegebenenfalls substituiertes Alkyl, Alkenyl, Alkinyl, Cycloalkyl, Aryl oder Alkoxycarbonyl steht,
mehrere Verfahren zu ihrer Herstellung sowie ihre Verwendung als Herbizide und Pflanzenwuchsregulatoren.

## N-(5-ALKYLTHIOPHENYL)-STICKSTOFFHETEROCYCLEN

Die Erfindung betrifft neue N-(5-Alkylthiophenyl)-Stickstoffheterocyclen, mehrere Verfahren zu ihrer Herstellung und ihre Verwendung als Herbizide und Pflanzenwuchsregulatoren.

Es ist bereits bekannt, daß bestimmte N-Aryl-Stickstoffheterocyclen, wie beispielsweise das N-(2,4-Dichlor-5-isopropoxyphenyl)-3-isopropylidenbernsteinsäureimid herbizide Eigenschaften besitzen (vergl. EP-A 190 755 oder DE-OS 3 642 372).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Es wurden neue N-(5-Alkylthiophenyl)-Stickstoffheterocyclen der allgemeinen Formel (I)

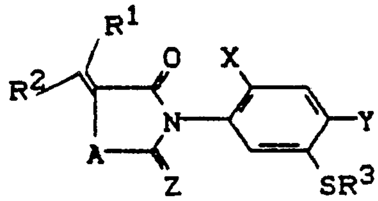

(I)

in welcher
$R^1$ und $R^2$ entweder unabhängig voneinander jeweils für Wasserstoff oder Alkyl stehen oder gemeinsam für einen zweifach verknüpften Alkandiylrest stehen,
$R^3$ für Wasserstoff, für jeweils gegebenenfalls substituiertes Alkyl, Alkenyl, Alkinyl, Cycloalkyl, Arylalkyl oder Heteroarylalkyl steht,
X für Wasserstoff oder Halogen steht,
Y für Halogen oder Alkyl steht,
Z für Sauerstoff oder Schwefel steht,
A für Sauerstoff, für eine $CH\text{-}R^4$-Gruppe, für eine

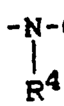-Gruppe oder für eine 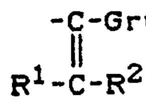-Gruppe steht,

wobei
$R^4$ für Wasserstoff, für jeweils gegebenenfalls substituiertes Alkyl, Alkenyl, Alkinyl, Cycloalkyl, Aryl oder Alkoxycarbonyl steht,
gefunden.

Weiterhin wurde gefunden, daß man die neuen N-(5-Alkylthiophenyl)-Stickstoffheterocyclen der allgemeinen Formel (I)

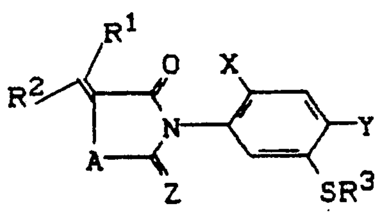

(I)

in welcher
$R^1$ und $R^2$ entweder unabhängig voneinander jeweils für Wasserstoff oder Alkyl stehen oder gemeinsam für einen zweifach verknüpften Alkandiylrest stehen,
$R^3$ für Wasserstoff, für jeweils gegebenenfalls substituiertes Alkyl, Alkenyl, Alkinyl, Cycloalkyl, Arylalkyl oder Heteroarylalkyl steht,
X für Wasserstoff oder Halogen steht,

Y für Halogen oder Alkyl steht,
Z für Sauerstoff oder Schwefel steht,
A für Sauerstoff, für eine CH-$R^4$-Gruppe, für eine

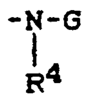-Gruppe oder für eine 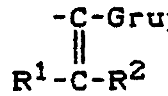-Gruppe steht,

wobei
$R^4$ für Wasserstoff, für jeweils gegebenenfalls substituiertes Alkyl, Alkenyl, Alkinyl, Cycloalkyl, Aryl oder Alkoxycarbonyl steht,
nach einem der im Folgenden beschriebenen Verfahren erhält:
a) Man erhält N-(5-Alkylthiophenyl)-Stickstoffheterocyclen der Formel (Ia),

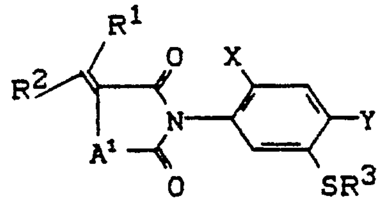

(Ia)

in welcher
$A^1$ für eine -CH-$R^4$-Gruppe oder für eine

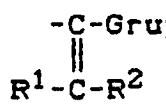-Gruppe

steht und
$R^1$, $R^2$, $R^3$, $R^4$, X und Y die oben angegebene Bedeutung haben,
wenn man Alkylidenbernsteinsäureanhydride der Formel (II),

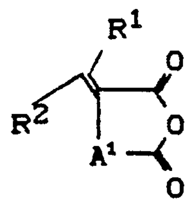

(II)

in welcher
$A^1$, $R^1$ und $R^2$ die oben angegebene Bedeutung haben,
mit Anilinen der Formel (III),

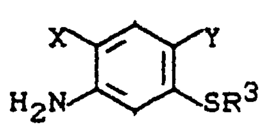

(III)

in welcher
X, Y und $R^3$ die oben angegebene Bedeutung haben,
gegebenenfalls in Gegenwart eines Verdünnungsmittels und gegebenenfalls in Gegenwart eines Reaktionshilfsmittels umsetzt;
b) Die nach Verfahren a) erhältlichen N-(5-Alkylthiophenyl)-Stickstoffheterocyclen der Formel (Ib)

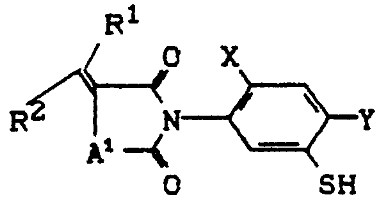

(Ib)

in welcher
$R^1$, $R^2$, X, Y und $A^1$ die oben angegebene Bedeutung haben,
werden mit Alkylierungsmitteln der Formel (IV),
$R^3$-$E^1$ (IV)
in welcher
$E^1$ für eine elektronenanziehende Abgangsgruppe steht und
$R^3$ die oben angegebene Bedeutung hat,
gegebenenfalls in Gegenwart eines Verdünnungsmittels und gegebenenfalls in Gegenwart eines Reaktionshilfsmittels umgesetzt;
c) Die nach Verfahren a) erhältlichen N-(5-Alkylthiophenyl)-Stickstoffheterocyclen der Formel (Ic),

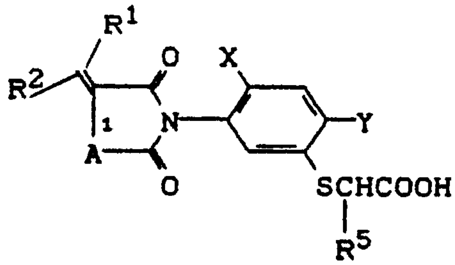

(Ic)

in welcher
$R^1$, $R^2$, X, Y und $A^1$ die oben angegebene Bedeutung haben und
$R^5$ für Wasserstoff, Halogen oder Alkyl steht,
werden mit Alkoholen der Formel (V),
$R^6OH$ (V)
in welcher
$R^6$ für jeweils gegebenenfalls substituiertes Alkyl, Alkenyl, Alkinyl oder Cycloalkyl steht,
gegebenenfalls in Gegenwart eines Verdünnungsmittels und gegebenenfalls in Anwesenheit einer Säure umgesetzt;
d) Man erhält N-(5-Alkylthiophenyl)-Stickstoffheterocyclen der Formel (Id),

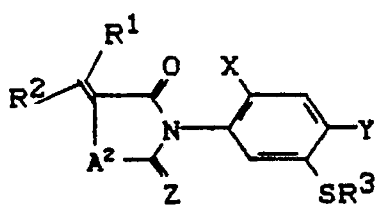

(Id)

in welcher
$R^1$, $R^2$, $R^3$, X, Y und Z die oben angegebene Bedeutung haben und
$A^2$ für eine

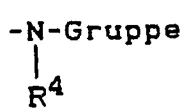

oder für Sauerstoff steht,
wobei $R^4$ die oben angegebene Bedeutung hat,
wenn man Carbonsäureester der Formel (VI),

$R^7$-$COOR^8$ (VI)

in welcher
$R^7$ für einen Rest der Formel

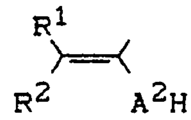
oder
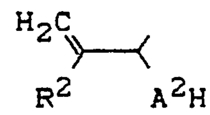
und

$R^8$ für Alkyl steht, wobei
$R^1$, $R^2$ und $A^2$ die oben angegebene Bedeutung haben,
oder deren Säureadditionssalze
mit Iso(thio)-cyanaten der Formel (VII)

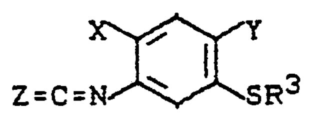
(VII)

in welcher
$R^3$, X, Y und Z die oben angegebene Bedeutung haben,
gegebenenfalls in Gegenwart eines Verdünnungsmittels und gegebenenfalls in Gegenwart eines Reaktionshilfsmittels umsetzt;
e) Die nach Verfahren (d) erhältlichen N-(5-Alkylthiophenyl)-Stickstoffheterocyclen der Formel (Ie)

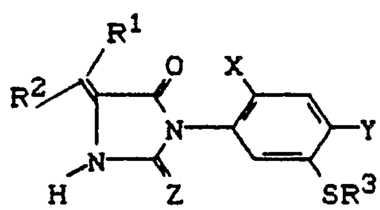
(Ie)

in welcher
$R^1$, $R^2$, $R^3$, X, Y und Z die oben angegebene Bedeutung haben,
werden mit Alkylierungsmitteln der Formel (VIII),

$R^{4-1}$-$E^2$ (VIII)

in welcher
$E^2$ für eine elektronenanziehende Abgangsgruppe steht und
$R^{4-1}$ für jeweils gegebenenfalls substituiertes Alkyl, Alkenyl, Alkinyl, Cycloalkyl oder Alkoxycarbonyl steht,
gegebenenfalls in Gegenwart eines Verdünnungsmittels und gegebenenfalls in Gegenwart eines Reaktionshilfsmittels umgesetzt.

Schließlich wurde gefunden, daß die neuen N-(5-Alkylthiophenyl)-Stickstoffheterocyclen der allgemeinen Formel (I) herbizide und pflanzenwuchsregulierende Eigenschaften besitzen.

Überraschenderweise zeigen die erfindungsgemäßen N-(5-Alkylthiophenyl)-Stickstoffheterocyclen der allgemeinen Formel (I) bei einer vergleichbaren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Technik bekannten N-Aryl-Stickstoffheterocyclen, wie beispielsweise das N-(2,4-Dichlor-5-isopropoxyphenyl)-3-isopropylidenbernsteinsäureimid, welches eine chemisch und wirkungsmäßig naheliegende Verbindung ist.

Die erfindungsgemäßen N-(5-Alkylthiophenyl)-Stickstoffheterocyclen sind durch die Formel (I) allgemein definiert.

Bevorzugt sind Verbindungen der Formel (I), bei welchen

$R^1$ und $R^2$ entweder unabhängig voneinander jeweils für Wasserstoff oder für geradkettiges oder verzweigtes Alkyl mit 1 bis 4 Kohlenstoffatomen stehen oder gemeinsam für einen zweifachverknüpften Alkandiylrest mit 2 bis 7 Kohlenstoffatomen stehen,

$R^3$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkylthiocarbonylalkyl, Dialkylaminocarbonylalkyl, Alkoxycarbonylalkyl, Alkoxycarbonylhalogenalkyl, Carboxyalkyl, Carboxyhalogenalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils in Frage kommen: Halogen sowie jeweils geradkettiges oder verzweigtes Alkyl oder Alkoxy mit jeweils 1 bis 4 Kohlenstoffatomen, $R^3$ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen Alkylteilen steht und schließlich für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten in Frage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

X für Wasserstoff, Fluor, Chlor oder Brom steht,

Y für Fluor, Chlor, Brom, Methyl oder Ethyl steht,

Z für Sauerstoff oder Schwefel steht,

A für Sauerstoff, für eine -CH-$R^4$-Gruppe, für eine

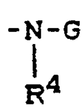 -Gruppe oder für eine 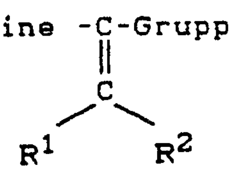 -Gruppe steht,

wobei

$R^4$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils in Frage kommen: Halogen sowie jeweils geradkettiges oder verzweigtes Alkyl oder Alkoxy mit jeweils 1 bis 4 Kohlenstoffatomen.

Besonders bevorzugt sind diejenigen Verbindungen der Formel (I), bei welchen

$R^1$ und $R^2$ entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen,

$R^3$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom, steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl, Alkylthiocarbonylalkyl, Dialkylaminocarbonylalkyl, Carboxyalkyl, Carboxyhalogenalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl und/oder Ethyl substituiertes Oxetanylmethyl, Oxetanylethyl, Tetrahydrofuranylmethyl Tetrahydrofuranylethyl, Tetrahydropyranylmethyl oder Tetrahydropyranylethyl steht, oder für jeweils gegebenenfalls ein- bis dreifach, gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

X für Wasserstoff, Fluor oder Chlor steht,

Y für Fluor, Chlor oder Brom steht,

Z für Sauerstoff oder Schwefel steht,

A für Sauerstoff, für eine -CH-$R^4$-Gruppe, für eine

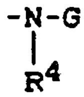-Gruppe oder für eine 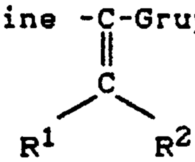-Gruppe steht,

wobei

$R^4$ für Wasserstoff, für Methyl, Ethyl, n- oder i-Propyl n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls einbis dreifach, gleich und verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, oder $R^4$ für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht.

Ganz besonders bevorzugt ist die Gruppe von Verbindungen der Formel (I), bei welchen

$R^1$ und $R^2$ entweder unabhängig voneinander jeweils für Wasserstoff oder Methyl oder gemeinsam für einen Ethan-1,2-diylrest, einen Butan-1,4-diylrest oder einen Pentan-1,5-diylrest stehen und

$R^3$ für Wasserstoff, Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl, Carboxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht oder für jeweils gegebenenfalls ein- bis dreifach, gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

X für Wasserstoff oder Fluor steht,

Y für Chlor oder Brom steht,
Z für Sauerstoff oder Schwefel steht,
A für Sauerstoff, für eine -CH-$R^4$-Gruppe, für eine

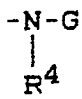 -Gruppe oder für eine 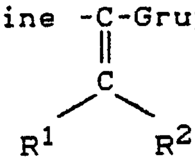 -Gruppe steht und

$R^4$ für Wasserstoff, Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht.

Ganz besonders bevorzugt ist die Gruppe von Verbindungen der Formel (I), in denen A für $CH_2$ steht und die übrigen Substituenten, die oben als bevorzugt, besonders bevorzugt oder ganz besonders bevorzugt angegebenen Bedeutungen haben.

Insbesondere hervorgehoben seien die Verbindungen der Formel (I), bei welcher
$R^1$ und $R^2$ unabhängig voneinander jeweils für Wasserstoff oder Methyl stehen,
$R^3$ für Wasserstoff, Methyl, Ethyl, n- oder i-Propyl, n-, i-, s- oder t-Butyl, Allyl, Propargyl, jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor oder Chlor, jeweils geradkettiges oder verzweigtes Cyanalkyl, Alkoxyalkyl, Carboxyalkyl, Alkoxycarbonylalkyl oder Alkoxyalkoxycarbonylalkyl mit jeweils 1 bis 4 Kohlenstoffatomen in den einzelnen Alkylteilen steht,
X für Wasserstoff oder Fluor steht,
Y für Chlor oder Brom steht,
Z für Sauerstoff steht und
A für $CH_2$, $C = C(CH_3)_2$, NH oder $NCH_3$ steht.

Beispiele für die erfindungsgemäßen Verbindungen der Formel (I) sind in der nachstehenden Tabelle 1 aufgeführt - vergl. auch die Herstellungsbeispiele.

Tabelle 1: Beispiele für die Verbindungen der Formel (I)

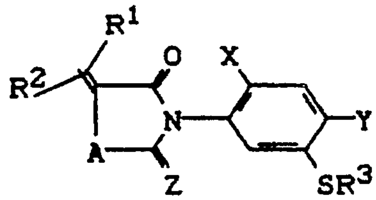

(I)

| $R^1$ | $R^2$ | $R^3$ | X | Y | Z | A |
|---|---|---|---|---|---|---|
| H | H | H | F | Cl | O | $CH_2$ |
| H | H | H | F | Br | O | $CH_2$ |
| H | H | H | H | Cl | O | $CH_2$ |
| H | H | $CH_3$ | F | Cl | O | $CH_2$ |
| H | H | $CH_2C{\equiv}C-H$ | F | Br | O | $CH_2$ |
| H | H | $CH_2CH{=}CH_2$ | F | Cl | O | $CH_2$ |
| $CH_3$ | H | $CH_2C{\equiv}C-H$ | F | Cl | O | $CH_2$ |
| H | H | $CH_2COOC_2H_5$ | F | Cl | O | $CH_2$ |
| H | H | $CH(CH_3)COOC_2H_5$ | F | Cl | O | $CH_2$ |
| H | H | $CH_2CN$ | F | Br | O | $CH_2$ |
| $CH_3$ | H | $CH(CH_3)CN$ | F | Cl | O | $CH_2$ |
| $CH_3$ | H | $CH_2OCH_3$ | F | Cl | O | $CH_2$ |
| $CH_3$ | H | $CH_2$ 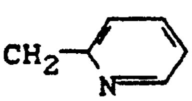 | F | Cl | O | $CH_2$ |
| H | H | $CHCOOC_2H_5$ with $CH_3$ (branch) | F | Br | O | $CH_2$ |
| $CH_3$ | $CH_3$ | $CH(CH_3)C{\equiv}CH$ | F | Cl | O | $CH_2$ |

Tabelle 1: Fortsetzung

| $R^1$ | $R^2$ | $R^3$ | X | Y | Z | A |
|---|---|---|---|---|---|---|
| $CH_3$ | $CH_3$ | $CH_2CONHC_2H_5$ | F | Cl | O | $CH_2$ |
| $CH_3$ | $CH_3$ | $CHCON(CH_3)_2$<br>\|<br>$CH_3$ | F | Cl | O | $CH_2$ |
| $(CH_2)_4$ | | $CH_2C{\equiv}CH$ | F | Cl | O | $CH_2$ |
| $CH_3$ | H | H | F | Cl | O | $CH_2$ |
| $CH_3$ | H | H | F | Br | O | $CH_2$ |
| H | H | $CH_3$ | F | Br | O | $CH_2$ |
| $CH_3$ | $CH_3$ | $CH(CH_3)COOC_2H_5$ | F | Cl | O | $=C(CH_3)_2$ |
| $CH_3$ | $CH_3$ | $CH_2C{\equiv}CH$ | F | Cl | O | $CHCH_3$ |
| $CH_3$ | $CH_3$ | $CH(CH_3)C{\equiv}CH$ | H | Br | O | $CH_2$ |
| $CH_3$ | $CH_3$ | $CH_2CH{=}CH_2$ | F | Br | O | $CH_2$ |
| $CH_3$ | $CH_3$ | $CH(CH_3)COOCH_2CF_3$ | F | Cl | O | $CH_2$ |
| $CH_3$ | $CH_3$ | $CHCOOC_2H_5$<br>\|<br>$C_2H_5$ | F | Cl | O | $CH_2$ |
| $CH_3$ | $CH_3$ | $CH_2CN$ | F | Cl | O | $CH_2$ |
| $CH_3$ | $CH_3$ | $CH_2$ 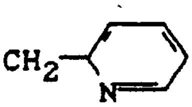 | F | Cl | O | $CH_2$ |
| $CH_3$ | $CH_3$ | $CHCOOC_2H_5$<br>\|<br>$CH_3$ | F | Br | O | $CH_2$ |

<u>Tabelle 1:</u> Fortsetzung

| $R^1$ | $R^2$ | $R^3$ | X | Y | Z | A |
|---|---|---|---|---|---|---|
| $CH_3$ | $CH_3$ | $CH_2OCH_3$ | F | Cl | O | $CH_2$ |
| $CH_3$ | H | $CH_2OC_2H_5$ | F | Cl | O | $CHCH_3$ |
| $CH_3$ | $CH_3$ | $CH_2C{\equiv}CH$ | F | Br | O | $CH_2$ |
| $CH_3$ | $CH_3$ | H | F | Br | O | $CH_2$ |
| $CH_3$ | $CH_3$ | $CHF_2$ | F | Cl | O | $CH_2$ |
| $CH_3$ | $CH_3$ | $C_2H_5$ | F | Cl | O | $CH_2$ |
| $CH_3$ | $CH_3$ | $CH(CH_3)_2$ | F | Cl | O | $CH_2$ |
| $CH_3$ | $CH_3$ | $CH_2OCH_3$ | F | Br | O | $CH_2$ |
| $CH_3$ | $CH_3$ | $CH_2C{\equiv}CH$ | H | Cl | O | $CH_2$ |
| $CH_3$ | $CH_3$ | $CH_2\text{-}CH{=}CH_2$ | F | Br | O | $CHCH_3$ |
| $CH_3$ | $CH_3$ | $CH(CH_3)C{\equiv}CH$ | F | Br | O | $CH_2$ |
| $CH_3$ | $CH_3$ | $CH_2COOC_2H_5$ | F | Cl | O | $CH_2$ |
| $CH_3$ | $CH_3$ | $CH(C_3H_7)COOC_2H_5$ | F | Cl | O | $CH_2$ |
| $CH_3$ | $CH_3$ | $CH_2CN$ | F | Br | O | $CHCH_3$ |
| $CH_3$ | $CH_3$ | $CHCN$<br>$CH_3$ | F | Cl | O | $CH_2$ |
| $CH_3$ | $CH_3$ | $CH_2$ 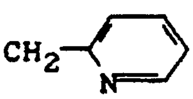 | F | Br | O | $CH_2$ |
| $CH_3$ | $CH_3$ | $CHCOOCH_2C{\equiv}CH$<br>$CH_3$ | F | Cl | O | $CH_2$ |

Tabelle 1: Fortsetzung

| $R^1$ | $R^2$ | $R^3$ | X | Y | Z | A |
|---|---|---|---|---|---|---|
| $CH_3$ | $CH_3$ | $CH_2CONHCH_3$ | F | Cl | O | $CH_2$ |
| $CH_3$ | $CH_3$ | $CHCOSC_2H_5$<br>$CH_3$ | F | Cl | O | $CH_2$ |
| $CH_3$ | $CH_3$ | $CH_2CN$ | F | Cl | O | $CHCH_3$ |
| $CH_3$ | $CH_3$ | $CH(CH_3)COOH$ | F | Br | O | $CH_2$ |
| $CH_3$ | $CH_3$ | $CH_3$ | F | Br | O | $CH_2$ |
| $C_2H_5$ | $CH_3$ | $CH_2C{\equiv}CH$ | F | Cl | O | $CH_2$ |
| $CH_3$ | $CH_3$ | $CH(CH_3)C{\equiv}CH$ | F | - | O | $CH_2$ |
| $CH_3$ | $CH_3$ | $CH_2CHClCH_2Cl$ | F | Cl | O | $CH_2$ |
| $CH_3$ | $CH_3$ | $CH_2COOCH_2OC_2H_5$ | F | Br | O | $CH_2$ |
| $CH_3$ | $CH_3$ | $CH_2COO$ 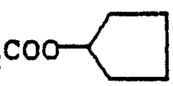 | F | Cl | O | $CH_2$ |
| $CH_3$ | $CH_3$ | $CH_2COO$ 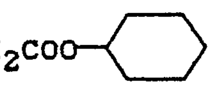 | F | Cl | O | $CH_2$ |
| $CH_3$ | $CH_3$ | $CHCOOCH_2CH_2Cl$<br>$CH_3$ | F | Br | O | $CH_2$ |
| $CH_3$ | $CH_3$ | $CHFCOOC_2H_5$ | F | Br | O | $CH_2$ |
| $CH_3$ | $CH_3$ | $CH_2OC_2H_5$ | F | Br | O | $CH_2$ |
| $CH_3$ | $CH_3$ | $CH_2C{\equiv}CH$ | F | Cl | O | $NC_2H_5$ |
| $CH_3$ | $CH_3$ | $CH_2C{\equiv}CH$ | F | Cl | O | NH |
| $CH_3$ | $CH_3$ | $CH(CH_3)C{\equiv}CH$ | F | Cl | O | NH |

<u>Tabelle 1:</u> Fortsetzung

| $R^1$ | $R^2$ | $R^3$ | X | Y | Z | A |
|---|---|---|---|---|---|---|
| $CH_3$ | $CH_3$ | $CH_2C\equiv CH$ | F | Cl | O | $NCH_3$ |
| $CH_3$ | $CH_3$ | $CH(CH_3)C\equiv CH$ | F | Cl | O | $NCH_3$ |
| $CH_3$ | $CH_3$ | $CH_2\underset{H}{C}=CH_2$ | F | Cl | O | $NCH_3$ |
| $CH_3$ | $CH_3$ | $CH_2\underset{H}{C}=CH_2$ | F | Br | O | NH |
| $CH_3$ | $CH_3$ | $CH_2\underset{H}{C}=CH_2$ | F | Br | O | $NCH_3$ |
| $CH_3$ | $CH_3$ | $CH_2COOC_3H_7$ | F | Br | O | NH |
| $CH_3$ | $CH_3$ | $CH_2COOC_2H_5$ | F | Br | O | $NCH_3$ |
| $CH_3$ | $CH_3$ | $CH_2CN$ | F | Cl | O | NH |
| $CH_3$ | $CH_3$ | $CH_2CN$ | F | Cl | O | $NCH_3$ |
| $CH_3$ | $CH_3$ | $\underset{CH_3}{\underset{\vert}{C}}HCOOCH_3$ | F | Cl | O | NH |
| $CH_3$ | $CH_3$ | $\underset{CH_3}{\underset{\vert}{C}}HCOOC_2H_5$ | F | Cl | O | $NCH_3$ |
| $CH_3$ | $CH_3$ | $\underset{CH_3}{\underset{\vert}{C}}HCOOC_2H_5$ | F | Br | O | $NC_2H_5$ |
| $CH_3$ | $CH_3$ | $CH_2C\equiv CH$ | F | Br | O | NH |
| $CH_3$ | $CH_3$ | $CH(CH_3)C\equiv CH$ | F | Cl | S | NH |
| $CH_3$ | $CH_3$ | $CH_2C\equiv CH$ | F | Br | O | $NCH_3$ |
| $CH_3$ | $CH_3$ | $CH(CH_3)C\equiv CH$ | F | Cl | S | $NCH_3$ |
| $CH_3$ | $CH_3$ | $CH_2C\equiv CH$ | F | Cl | O | $NCHF_2$ |

<u>Tabelle 1:</u> Fortsetzung

| $R^1$ | $R^2$ | $R^3$ | X | Y | Z | A |
|---|---|---|---|---|---|---|
| $CH_3$ | $CH_3$ | $CH(CH_3)C≡CH$ | F | Br | O | $NCH_3$ |
| $CH_3$ | $CH_3$ | $CH_2C≡CH$ | F | Cl | S | $NC_2H_5$ |
| $CH_3$ | $CH_3$ | $CH_2C=CH_2$<br>H | F | Br | O | $NCOOC_2H_5$ |
| $CH_3$ | $CH_3$ | $CH_2C=CH_2$<br>H | F | Cl | O | $NCHF_2$ |
| $CH_3$ | $CH_3$ | $CH_2C=CH_2$<br>H | F | Cl | O | $NC_2H_5$ |
| $CH_3$ | $CH_3$ | $CH_2COO$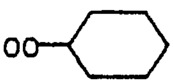 | F | Br | O | NH |
| $CH_3$ | $CH_3$ | CHCOO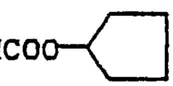<br>$CH_3$ | F | Cl | O | $NCH_3$ |
| $CH_3$ | $CH_3$ | $CH_2CN$ | F | Br | O | $NCH_3$ |
| $CH_3$ | $CH_3$ | CHCN<br>$CH_3$ | F | Cl | O | $NCH_3$ |
| $CH_3$ | $CH_3$ | $CH_2CH_2OC_2H_5$ | F | Cl | O | $NCH_2$ |
| $CH_3$ | $CH_3$ | $CH_2C≡CH$ | H | Cl | O | O |
| $CH_3$ | $CH_3$ | $CH(CH_3)C≡CH$ | H | Cl | O | O |
| $CH_3$ | $CH_3$ | $CH_2C≡CH$ | H | Cl | S | O |
| $CH_3$ | $CH_3$ | $CH_2C≡CH$ | F | Cl | O | O |
| $CH_3$ | $CH_3$ | $CH_2C≡CH$ | F | Cl | S | O |
| $CH_3$ | $CH_3$ | $CH(CH_3)C≡CH$ | F | Cl | O | O |

<u>Tabelle 1:</u> Fortsetzung

| $R^1$ | $R^2$ | $R^3$ | X | Y | Z | A |
|---|---|---|---|---|---|---|
| $CH_3$ | $CH_3$ | $CH_2C{\equiv}CH$ | H | Br | S | O |
| $CH_3$ | $CH_3$ | $CH(CH_3)C{\equiv}CH$ | F | Br | O | O |
| $CH_3$ | $CH_3$ | $CH_2C{\equiv}CH$ | F | Br | S | O |
| $CH_3$ | $CH_3$ | $CH_2\underset{H}{C}=CH_2$ | H | Cl | O | O |
| $CH_3$ | $CH_3$ | $CH_2\underset{H}{C}=CH_2$ | F | Cl | O | O |
| $CH_3$ | $CH_3$ | $CH_2\underset{H}{C}=CH_2$ | H | Br | O | O |
| $CH_3$ | $CH_3$ | $CH_2\underset{H}{C}=CH_2$ | F | Br | O | O |
| $CH_3$ | $CH_3$ | $CH_2\underset{H}{C}=CH_2$ | H | Cl | S | O |
| $CH_3$ | $CH_3$ | $CH_2\underset{H}{C}=CH_2$ | F | Cl | S | O |
| $CH_3$ | $CH_3$ | $CH_2COOC_2H_5$ | F | Br | O | O |
| $CH_3$ | $CH_3$ | $CH_2COOC_2H_5$ | F | Cl | S | O |
| $CH_3$ | $CH_3$ | $\underset{CH_3}{\underset{\vert}{CH}}COOC_2H_5$ | F | Cl | O | O |
| $CH_3$ | $CH_3$ | $CH_2CN$ | F | Br | O | O |
| $CH_3$ | $CH_3$ | $CH(CH_3)CN$ | F | Cl | O | O |
| $CH_3$ | $CH_3$ | $CH_2COO$ 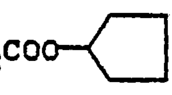 | F | Cl | O | O |
| $CH_3$ | $CH_3$ | $CH(CH_3)_2$ | F | Cl | O | O |

Tabelle 1: Fortsetzung

| $R^1$ | $R^2$ | $R^3$ | X | Y | Z | A |
|---|---|---|---|---|---|---|
| $CH_3$ | $CH_3$ | $CH_2COO$ 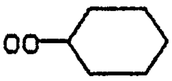 | F | Br | O | O |
| $CH_3$ | $CH_3$ | $CH(CH_3)_2$ | F | Br | O | O |
| $CH_3$ | $CH_3$ | $CH_2COOCH_2OC_2H_5$ | F | Cl | S | O |
| $CH_3$ | $CH_3$ | $CH_2COOC_3H_7$ | F | Cl | O | O |
| $CH_3$ | $CH_3$ | $CH(C_2H_5)COOC_2H_5$ | F | Cl | O | O |
| $CH_3$ | $CH_3$ | $CH(CH_3)COOCH_2CF_3$ | F | Cl | O | O |
| $CH_3$ | $CH_3$ | $CH_2OCH_3$ | F | Cl | O | O |
| $CH_3$ | $CH_3$ | $CH_2OC_2H_5$ | F | Br | O | O |
| $CH_3$ | $CH_3$ | $CH_2$ 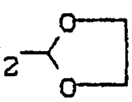 | F | Cl | O | O |

Verwendet man für das erfindungsgemäße Verfahren (a) beispielsweise 2-Fluor-4-chlor-5-allylthioanilin und Isopropylidenbernsteinsäureanhydrid als Ausgangsstoffe, so kann der Reaktionsverlauf durch das folgende Formelschema wiedergegeben werden:

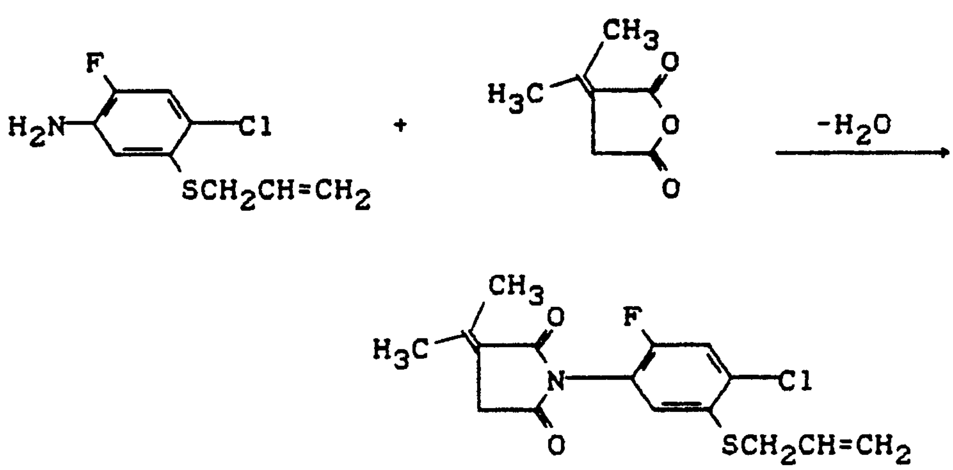


Verwendet man für das erfindungsgemäße Verfahren (b) beispielsweise 1-(4-chlor-5-sulfhydrylphenyl)-3-isopropylidenbernsteinsäureimid und Propargylbromid als Ausgangsstoffe, so kann der Reaktionsverlauf durch das folgende Formelschema wiedergegeben werden:

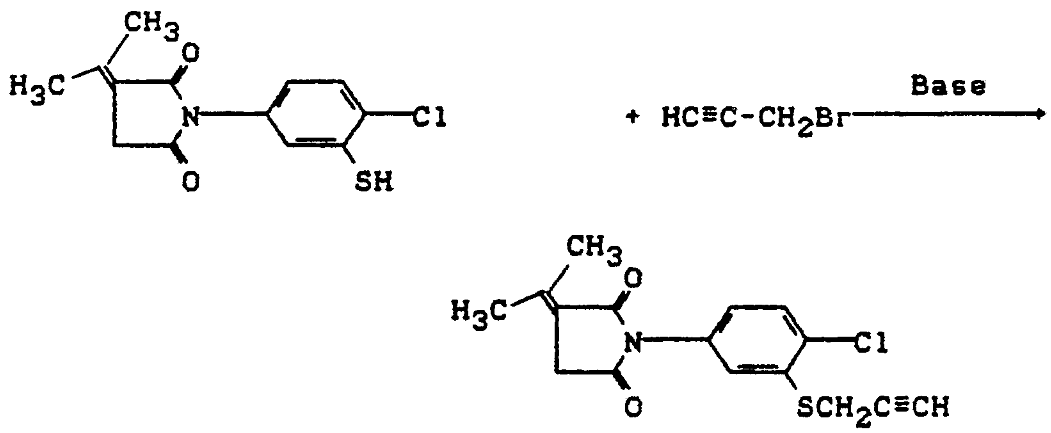


Verwendet man für das erfindungsgemäße Verfahren (c) beispielsweise 1-(4-Chlor-2-fluor-5-carboxymethylphenyl)-3-isopropylidenbernsteinsäureimid und Isopropanol als Ausgangsstoffe, so kann der Reaktionsverlauf durch das folgende Formelschema wiedergegeben werden:

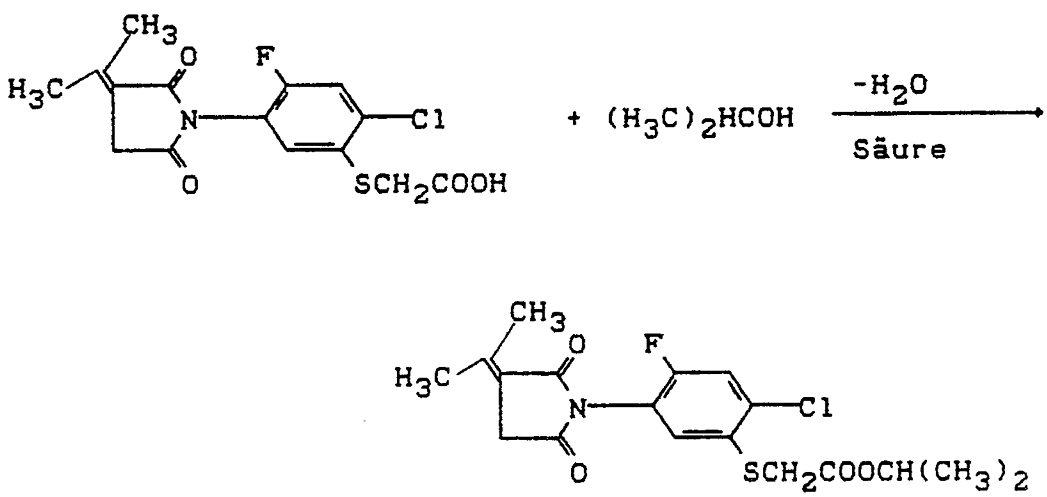


Verwendet man für das erfindungsgemäße Verfahren (d) beispielsweise 1-Amino-2,2-dimethylacrylsäureethylester und 4-Brom-2-Fluor-5-isopropylthiophenylisocyanat als Ausgangsstoffe, so kann der Reaktionsverlauf durch das folgende Formelschema wiedergegeben werden:

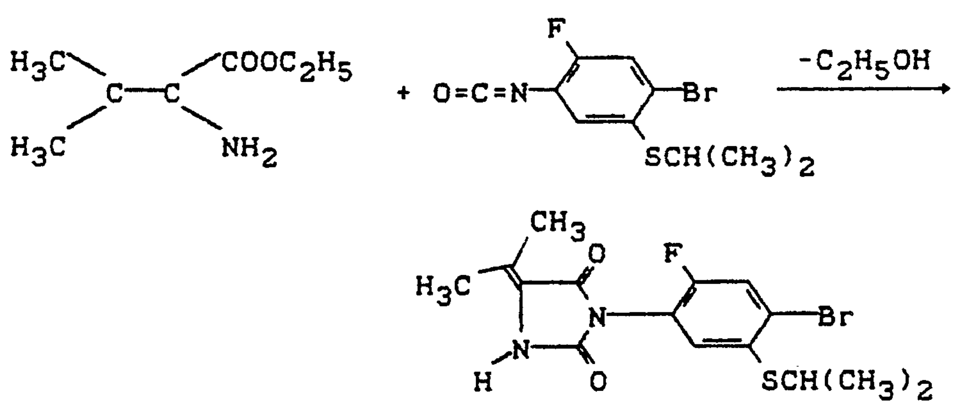


Verwendet man für das erfindungsgemäße Verfahren (d) beispielsweise 2-Hydroxy-3-methyl-3-butensäuremethylester und 4-Chlor-5-ethylthiophenylisocyanat als Ausgangsstoffe, so kann der Reaktionsverlauf durch das folgende Formelschema wiedergegeben werden:

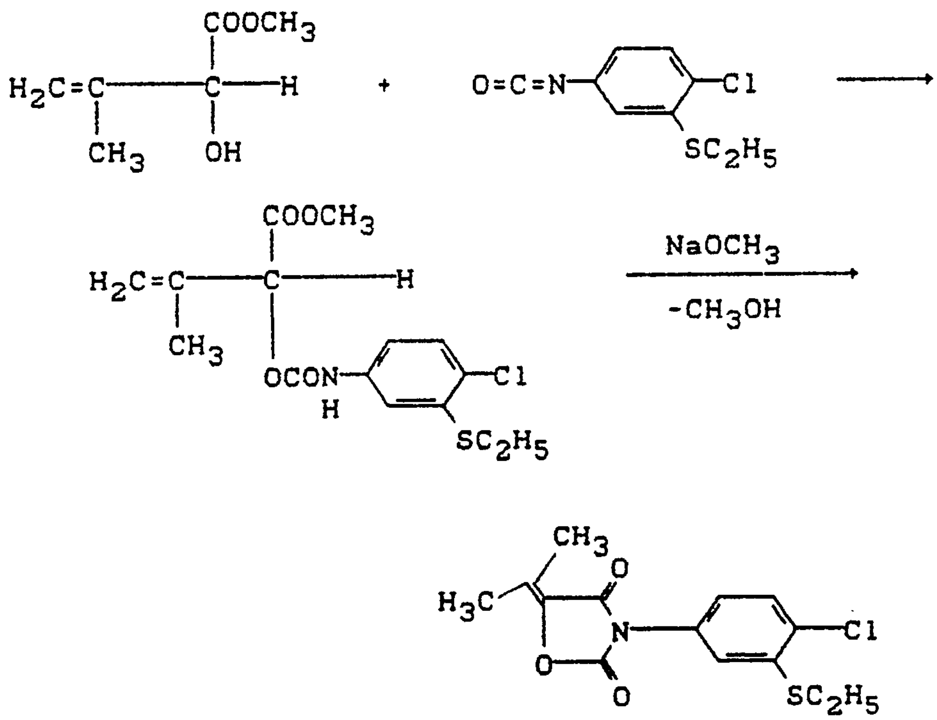


Verwendet man für das erfindungsgemäße Verfahren (e) beispielsweise 3-(4-Brom-2-fluor-5-alkylthiophenyl)-5-isopropylidenimidazol-2,4-dion und Methyliodid als Ausgangsstoffe, so kann der Reaktionsverlauf durch das folgende Formelschema wiedergegeben werden:

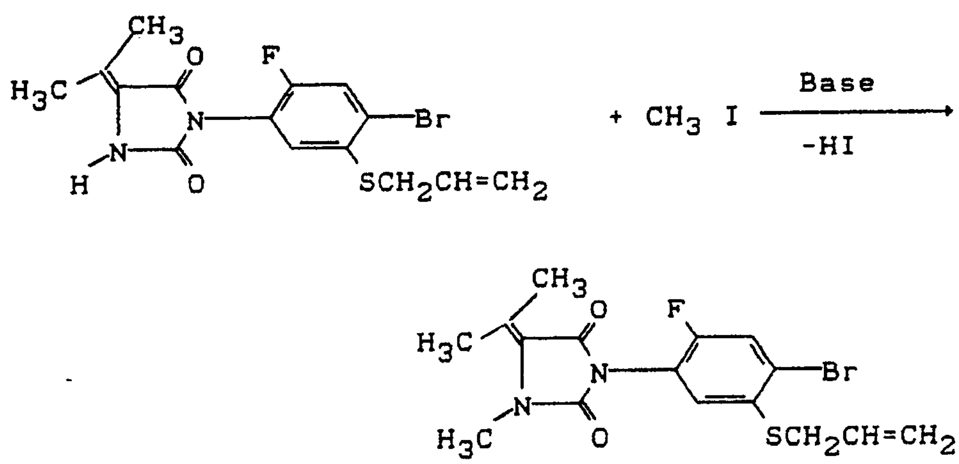


Die zur Durchführung des erfindungsgemäßen Verfahrens (a) als Ausgangsstoffe benötigten Alkylidenbernsteinsäureanhydride sind durch die Formel (II) allgemein definiert. In dieser Formel (II) steht $A^1$ vorzugsweise für eine -CH-$R^4$-Gruppe oder für

eine 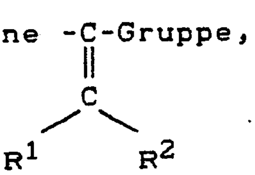

wobei
$R^1$, $R^2$ und $R^4$ vorzugsweise diejenigen Reste bedeuten, die bereits in Zusammenhang mit der Beschreibung der erfindungsgemäßen Stoffe der Formel (I) als bevorzugt für diese Substituenten genannt wurden.

Die Anhydride der Formel (II) sind bekannt oder erhältlich in Analogie zu bekannten Verfahren (vergl. z.B. Tetrahedron 25 , 4099 - 4108 [1969]; Liebigs Ann. Chem. 461 , 191).

Die zur Durchführung des erfindungsgemäßen Verfahrens (a) weiterhin als Ausgangsstoffe benötigten Aniline sind durch die Formel (III) allgemein definiert. In dieser Formel (III) stehen $R^3$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Aniline der Formel (III) sind bekannt oder erhältlich in Analogie zu bekannten Verfahren (vergl. z.B. EP-A 126 419; EP-A 142 648);

Die zur Durchführung des erfindungsgemäßen Verfahrens (b) als Ausgangsstoffe benötigten N-Aryl-Stickstoffheterocyclen sind durch die Formel (Ib) allgemein definiert. In dieser Formel (Ib) stehen $R^1$, $R^2$, X, Y und $A^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (Ib) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

$E^1$ steht für einen bei Alkylierungsmitteln üblichen Abgangsrest, vorzugsweise für Halogen, insbesondere für Chlor, Brom oder Iod, oder für jeweils gegebenenfalls substituiertes Alkylsulfonyloxy mit vorzugsweise 1 bis 4 Kohlenstoffatomen, Alkoxysulfonyloxy mit vorzugsweise 1 bis 4 Kohlenstoffatomen oder Arylsulfonyloxy mit vorzugsweise 6 bis 10 Kohlenstoffatomen, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten N-(5-Alkylthiophenyl)-Stickstoffheterocyclen sind durch die Formel (Ic) allgemein definiert. In dieser Formel (Ic) stehen $R^1$, $R^2$, X, Y und $A^1$ vorzugsweise für diejenigen Reste, die bereits im Zusammenhang mit der Beschreibung der erfindungsgemäßen Stoffe der Formel (I) als bevorzugt für diese Substituenten genannt wurden.

$R^5$ steht vorzugsweise für Wasserstoff, Fluor, Chlor, Methyl, Ethyl n- oder i-Propyl.

Die N-(5-Alkylthiophenyl)-Stickstoffheterocyclen der Formel (Ic) sind erfindungsgemäße Verbindungen und erhältlich mit Hilfe des erfindungsgemäßen Verfahrens (a).

Die zur Durchführung des erfindungsgemäßen Verfahrens (c) weiterhin als Ausgangsstoffe benötigten Alkohole sind durch die Formel (V) allgemein definiert. In dieser Formel (V) steht $R^6$ vorzugsweise für jeweils geradkettiges oder verzweigtes Alkyl, Alkoxyalkyl oder Halogenalkyl mit jeweils 1 bis 8 Kohlenstoffatomen in den einzelnen Alkylteilen und gegebenenfalls 1 bis 9, insbesondere 1 bis 5, gleichen oder verschiedenen Halogenatomen.

Die Alkohole der Formel (V) sind allgemein bekannte Verbindungen der organischen Chemie.

Die zur Durchführung des erfindungsgemäßen Verfahrens (d) als Ausgangsstoffe benötigten Carbonsäureester sind durch die Formel (VI) allgemein definiert. In dieser Formel (VI) steht $R^7$ vorzugsweise für einen Rest der Formel

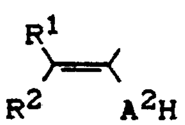

oder

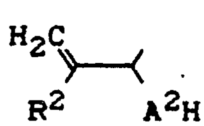

wobei

$R^1$, $R^2$ und $A^2$ vorzugsweise für diejenigen Reste stehen, die bereits im Zusammenhang mit der Beschreibung der erfindungsgemäßen Stoffe der Formel (I) als bevorzugt für diese Substituenten genannt wurden,

$R^8$ steht vorzugsweise für geradkettiges oder verzweigtes Alkyl mit 1 bis 4 Kohlenstoffatomen, insbesondere für Methyl oder Ethyl.

Die Carbonsäureester der Formel (VI) sind allgemein bekannte Verbindungen der organischen Chemie oder erhältlich in Analogie zu bekannten Verfahren (vergl. z.B. J. Org. Chem. 37 , 943 - 946 [1972]; EP-A-153 692; Chem. Pharm. Bull. 32 , 3934 - 3944 [1984]; Chem. Ber. 110 , 942 - 947 [1977]).

Die zur Durchführung des erfindungsgemäßen Verfahrens (d) weiterhin als Ausgangsstoffe benötigten

Iso(thio) cyanate sind durch die Formel (VII) allgemein definiert. In dieser Formel (VII) stehen $R^3$, X, Y und Z vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (VII) sind bekannt oder erhältlich in Analogie zu bekannten Verfahren (vergl. z.B. EP-A-304 920; EP-A-312 064; DE-A-3 604 645), beispielsweise, wenn man Aniline der Formel (III),

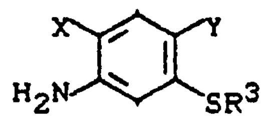

(III)

in welcher
$R^3$, X und Y die oben angegebene Bedeutung haben,
mit Phosgen, Thiophosgen oder Diphosgen ($Cl_3C$-O-CO-Cl) gegebenenfalls in Gegenwart eines Verdünnungsmittels wie beispielsweise Toluol bei Temperaturen zwischen 20° C und 120° C umsetzt.

Die zur Durchführung des erfindungsgemäßen Verfahrens (e) als Ausgangsstoffe benötigten N-Aryl-Stickstoffheterocyclen sind durch die Formel (Ie) allgemein definiert.

In dieser Formel (Ie) stehen $R^1$, $R^2$, $R^3$, X, Y und Z vorzugsweise für diejenigen Reste, die bereits im Zusammenhang mit der Beschreibung der erfindungsgemäßen Stoffe der Formel (I) als bevorzugt für diese Substituenten genannt wurden.

Die N-Aryl-Stickstoffheterocyclen der Formel (Id) sind erfindungsgemäße Verbindungen und erhältlich mit Hilfe des erfindungsgemäßen Verfahrens (d).

Die zur Durchführung des erfindungsgemäßen Verfahrens (e) weiterhin als Ausgangsstoffe benötigten Alkylierungsmittel sind durch die Formel (VIII) allgemein definiert. In dieser Formel (VIII) steht $R^{4-1}$ mit Ausnahme von Wasserstoff vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy mit vorzugsweise 1 bis 4 Kohlenstoffatomen, Alkoxysulfonyloxy mit vorzugsweise 1 bis 4 Kohlenstoffatomen oder Arylsulfonyloxy mit vorzugsweise 6 bis 10 Kohlenstoffatomen, wie insbesondere Methansulfonyloxy, Trifluormethansulfonyloxy, Methoxysulfonyloxy, Ethoxysulfonyloxy oder p-Toluolsulfonyloxy.

Die Alkylierungsmittel der Formel (VIII) sind allgemein bekannte Verbindungen der organischen Chemie.

Als Verdünnungsmittel zur Durchführung des erfindungsgemäßen Verfahrens (a) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren, wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich, andere übliche wasserabspaltende Mittel, wie beispielsweise N,N′-Dicyclohexylcarbodiimid oder übliche Acylierungskatalysatoren, wie beispielsweise 4-(N,N-Dimethylamino)pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20° C und 180° C, vorzugsweise bei Temperaturen zwischen 50° C und 150° C.

Zur Durchführung des erfindungsgemäßen Verfahrens (a) setzt man pro Mol an Alkylidenbernsteinsäureanhydrid der Formel (II) im allgemeinen 1 bis 1,5 Mol, vorzugsweise 1 bis 1,2 Mol an Anilin der Formel (III) und gegebenenfalls 0,01 bis 1,2 Mol, vorzugsweise 0,1 bis 1 Mol an Reaktionshilfsmittel ein. Die Reaktionsdurchführung, Aufarbeitung und Isolierung der Reaktionsprodukte erfolgt nach allgemein üblichen Methoden (vergl. auch die Herstellungsbeispiele).

Zur Durchführung des erfindungsgemäßen Verfahrens (b) kommen als Verdünnungsmittel inerte organische Lösungsmittel in Frage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylie rungsmittel der Formel (IV) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (b) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man zwischen -20° C und +150° C, vorzugsweise zwischen 0° C und +100° C.

Zur Durchführung des erfindungsgemäßen Verfahrens (b) setzt man pro Mol N-Aryl-Stickstoffheterocyclus der Formel (Ib) im allgemeinen jeweils 1 bis 20 Mol, vorzugsweise jeweils 1 bis 2 Mol, an Alkylierungsmittel der Formel (VI) und gegebenenfalls 1 bis 3 Mol, vorzugsweise 1 bis 2 Mol an Reaktionshilfsmittel, ein. Die Reaktionsdurchführung, Aufarbeitung und Isolierung der Reaktionsprodukte der Formel (I) erfolgt nach üblichen Methoden.

Als Verdünnungsmittel zur Durchführung des erfindungsgemäßen Verfahrens (c) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Auch kann der Alkohol der Formel (V) gleichzeitig als Lösungsmittel verwendet werden.

Das erfindungsgemäße Verfahren (c) wird vorzugsweise in Gegenwart eines geeigneten Reaktionshilfsmittels durchgeführt. Als solche kommen alle üblicherweise verwendbaren anorganischen und organischen Säuren in Frage.

Die Reaktionstemperaturen können bei der Durchführung des erfindungsgemäßen Verfahrens (c) in einem größeren Bereich variiert werden. Im allgemeinen arbeitet man bei Temperaturen zwischen 0° C und 150° C, vorzugsweise bei Temperaturen zwischen 20° C und 120° C.

Zur Durchführung des erfindungsgemäßen Verfahrens (c) setzt man pro Mol an N-Aryl-Stickstoffheterocyclus der Formel (Ic) im allgemeinen 1 bis 3 Mol, vorzugsweise 1 bis 1,5 Mol an Alkohol der Formel (V) und 0,1 bis 3 Mol, vorzugsweise 1 bis 2 Mol an Reaktionshilfsmittel ein.

Die Reaktionsdurchführung, Aufarbeitung und Isolierung der Reaktionsprodukte erfolgt nach allgemein üblichen Methoden (vergl. auch die Herstellungsbeispiele).

Als Verdünnungsmittel zur Durchführung des erfindungsgemäßen Verfahrens (d) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid.

Das erfindungsgemäße Verfahren (d) wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen 0° C und 180° C, vorzugsweise bei Temperaturen zwischen 20° C und 150° C.

Zur Durchführung des erfindungsgemäßen Verfahrens (d) setzt man pro Mol an Carbonsäureester der Formel (VI) oder eines entsprechenden Säureadditionssalzes im allgemeinen 0,5 bis 5 Mol, vorzugsweise 0,8 bis 1,5 Mol an Iso(thio)cyanat der Formel (VII) und gegebenenfalls 1 bis 10 Mol, vorzugsweise 1 bis 5 Mol an Reaktionshilfsmittel ein. Dabei ist es auch möglich, die Iso(thio)cyanate der Formel (VII) in einer vorgelagerten Reaktion aus Aminen der Formel (III) und Phosgen, Thiophosgen oder Diphosgen ($Cl_3C$-O-CO-Cl) direkt im Reaktionsgefäß herzustellen und ohne Isolierung im "Eintopfverfahren" mit den Carbonsäureestern der Formel (VI) weiter umzusetzen.

Die Reaktionsdurchführung, Aufarbeitung und Isolierung der Reaktionsprodukte erfolgt nach allgemein üblichen Methoden (vergl. auch die Herstellungsbeispiele).

Zur Durchführung des erfindungsgemäßen Verfahrens (e) kommen als Verdünnungsmittel inerte organische Lösungs mittel in Frage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylierungsmittel der Formel (VIII) in flüssiger Form, so ist es möglich, diese in entsprechendem Überschuß gleichzeitig als Verdünnungsmittel einzusetzen.

Als Reaktionshilfsmittel zur Durchführung des erfindungsgemäßen Verfahrens (e) kommen alle üblicherweise verwendbaren anorganischen oder organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (e) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (e) setzt man pro Mol N-Aryl-Stickstoffheterocyclus der Formel (Ie) im allgemeinen jeweils 1 bis 20 Mol, vorzugsweise jeweils 1 bis 15 Mol, an Alkylierungsmittel der Formel (VIII) und gegebenenfalls 1 bis 3 Mol, vorzugsweise 1 bis 2 Mol an Reaktionshilfsmittel, ein. Die Reaktionsdurchführung, Aufarbeitung und Isolierung der Reaktionsprodukte der Formel (I) erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xan thium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen sowohl im Vorauflauf- als auch im Nachauflauf-Verfahren.

In gewissem Umfang zeigen die Verbindungen der Formel (I) auch fungizide Wirkung, z.B. gegen Pyricularia oryzae an Reis, gegen Venturia-Arten und gegen Phytophtora-Arten.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in

Frage; ferner auch 2,4-D, 2,4-DB, 2,4-DP, Acifluorfen, Alachlor, Alloxydim, Atrazin, Bensulfuron, Bentazon, Bifenox, Bromoxynil, Chloridazon, Chlorimuron, Chlorsulfuron, Chlortoluron, Cinmethylin, Clopyralid, Cyanazin, Dichlofop, Dimethazone, EPTC = Eptame, Ethiozin, Fenoxaprop, Fluazifop, Fluometuron, Fluridone, Fluroxypyr, Fomesafen, Haloxyfop, Hexazinone, Imazamethabenz, Imazaquin, Imazethapyr, Ioxynil, Isoproturon, Lactofen, MCPA, MCPP, Metazachlor, Metolachlor, Metsulfuron, Norflurazon, Oryzalin, Oxyfluorfen, Pendimethalin, Pyridate, Quizalofopethyl, Sethoxydim, Simazin, Simetryne, Terbutryne, Thiameturon, Triallate, Trifluralin. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,005 und 5 kg Wirkstoff pro Hektar Bodenfläche, vorzugsweise zwischen 0,01 und 1 kg pro ha.

Die Herstellung und die Verwendung der erfindungsgemäßen Wirkstoffe geht aus den nachfolgenden Beispielen hervor.

## Herstellungsbeispiele

### Beispiel 1

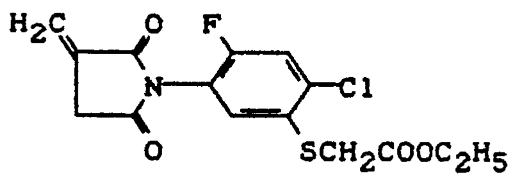

Verfahren (a)

Ein Gemisch aus 2,35 g (0,021 Mol) Methylenbernsteinsäureanhydrid, 5,27 g (0,02 Mol) 4-Chlor-2-fluor-5-ethoxycarbonylmethylthioanilin und 60 ml Eisessig wird 5 Stunden bei 135 °C gerührt. Nach Abkühlen und Einengen wird der Rückstand säulenchromatografisch aufgetrennt. Man erhält 2,14 g (29,9 % d. Th.) 1-(4-Chlor-2-fluor-4-ethoxycarbonylmethylthiophenyl)-3-methylenbernsteinsäureimid als Öl.

$^{1}$H-NMR (80 Mhz, $CDCl_3$, δ): 7,44 (d, 1H, J=8,0 Hz); 7,35 (d, 1H, J=9,0 Hz); 6,49 (t, 1H, J=1,0 Hz); 5,78 (t, 1H, J=1,0 Hz); 4,17 (g, 2H, J=8,0 Hz); 3,64 (s, 2H); 3,55 (bs, 2H); 1,22 (t, 3H, J=8,0 Hz).

### Beispiel 2

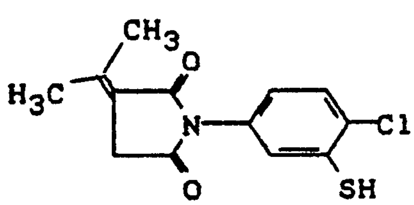

Verfahren (a)

3,19 g (0,02 Mol) 4-Chlor-5-sulfhydrylanilin, 2,91 g (0,021 Mol) Isopropylidenbernsteinsäureanhydrid, 0,3 g (0,015 Mol) Paratoluolsulfonsäure und 8 ml Toluol werden 4 Stunden bei 100 °C erhitzt. Man engt ein, versetzt mit 10 ml einer gesättigten Natriumhydrogencarbonatlösung und extrahiert mit Methylenchlorid. Nach Einengen und Säulenchromatografie erhält man 1,37 g (24,3 % d. Th.) 1-(4-Chlor-5-sulfhydrylphenyl)-3-isopropylidenbernsteinsäureimid vom Schmelzpunkt 146 - 148 °C.
$^1$H-NMR (80 Mhz, $CDCl_3$, δ): 7,45 (d, 1H, J = 8,0 Hz); 7,34 (d, 1H, J = 2,0 Hz); 7,07 (dd, 1H, J = 8,0 Hz, J = 2,0 Hz); 3,95 (s, 1H); 3,37 (s, 2H); 2,38 (s, 3H); 1,95 (s, 3H).

Beispiel 3

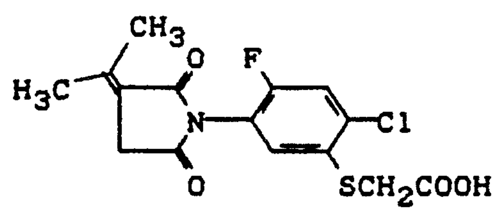

Verfahren (a)

Ein Gemisch aus 3,68 g (0,0263 Mol) Isopropylidenbernsteinsäureanhydrid, 5,89 g (0,025 Mol) 4-Chlor-2-fluor-5-carboxymethylthioanilin und 70 ml Eisessig erhitzt man 2 Stunden auf 135 °C. Nach Entfernung der flüchtigen Komponente versetzt man den Rückstand mit Diethylether, wobei das Produkt auskristallisiert. Nach Absaugen isoliert man 5,32 g (59,5 % d. Th) 1-(4-Chlor-2-fluor-5-carboxymethylthiophenyl)-3-isopropylidenbernsteinsäureimid vom Schmelzpunkt 144 - 147 °C.
$^1$H-NMR (80 Mhz, $CDCl_3$, δ): 7,41 (d, 1H, J = 8,0 Hz); 7,33 (d, 1H, J = 9,0 Hz): 6,47 (bs, 1H); 3,66 (9,2 H); 3,42 (s, 2H); 2,37 (s, 3H); 1,95 (s, 3H).

Beispiel 4

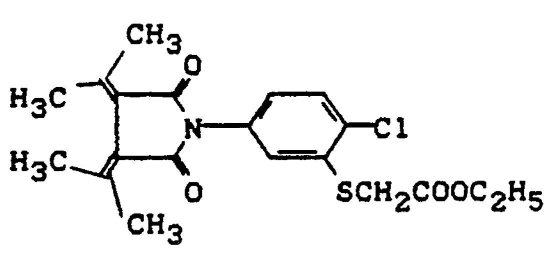

Verfahren (a)

Man erhitzt eine Lösung von 3,78 g (0,021 Mol) Diisopropylidenbernsteinsäureanhydrid und 4,91 g (0,02 Mol) 4-Chlor-5-ethoxycarbonylmethylthioanilin in 55 ml Eisessig fünf Stunden bei 135 °C. Nach Abkühlen engt man ein und versetzt das entstandene Bernsteinsäurehalbamid mit 2,4 g (0,03 Mol) Natriumacetat und 48 ml Essigsäureanhydrid. Man erhitzt das Reaktionsgemisch zwei Stunden bei 95 °C, kühlt ab und engt ein. Durch säulenchromatografische Auftrennung über Kieselgel erhält man 2,22 g (27,2 % d. Th.) 1-(4-Chlor-5-ethoxycarbonylmethylthiophenyl)-3,4-diisopropylidenbernsteinsäureimid vom Schmelzpunkt 119 - 123 °C.
$^1$H-NMR (80 Mhz, $CDCl_3$, δ): 7,45 (d, 1H, J = 8,0 Hz); 7,43 (d, 1H, J = 2,0 Hz); 7,19 (dd, 1H, J = 8,0 Hz, J = 2,0 Hz); 4,20 (g, 2H, J = 8,0Hz); 3,70 (s, 2H); 2,38 (s, 6H); 1,92 (s, 6H); 1,23 (t, 3H, J = 8,0 Hz).

Beispiel 5

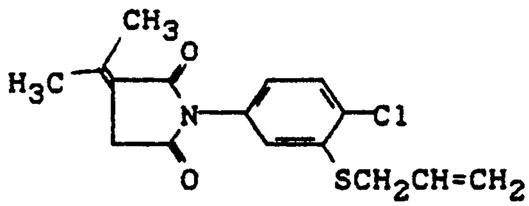

Verfahren (b)

Ein Gemisch aus 1,69 g (0,006 Mol) 1-(4-Chlor-5-sulfhydrylphenyl)-3-isopropylidenbernsteinsäureimid, 1,24 g (0,009 Mol) Kaliumcarbonat und 15 ml Acetonitril wird 1 Stunde bei 50 °C erhitzt. Man kühlt ab und tropft bei 25 °C 1,09 g (0,009 Mol) Allylbromid ein und erhitzt anschließend noch 2 Stunden bei 50 °C. Nach Einengen versetzt man den Rückstand mit Wasser und extrahiert mit Methylenchlorid. Die organische Phase wird über Natriumsulfat getrocknet, abfiltriert und eingeengt. Durch säulenchromatografische Reinigung über Kieselgel (Laufmittel: Ethylacetat-Petrolether Gradient) erhält man 0,63 g (32,7 % d. Th.) 1-(4-Chlor-5-allylthiophenyl)-3-isopropylidenbernsteinsäureimid vom Schmelzpunkt 86 - 91 °C.
$^1$H-NMR (80 Mhz, $CDCl_3$, δ): 7,44 (d, 1H, J = 8,0 Hz); 7,26 (d, 1H, J = 2,0 Hz); 7,10 (dd, 1H, J = 8,0 Hz, J = 2,0 Hz); 5,90 (m, 1H); 5,26 (dd, 1H, J = 1,65 Hz, J = 1,0 Hz); 5,16 (dd, 1H, J = 9,0 Hz, J = 1,0Hz); 3,59 (dd, 2H, J = 8,0 Hz, J = 1,0 Hz); 3,37 (s, 2H); 2,38 (s, 3H); 1,94 (s, 3H).

Beispiel 6

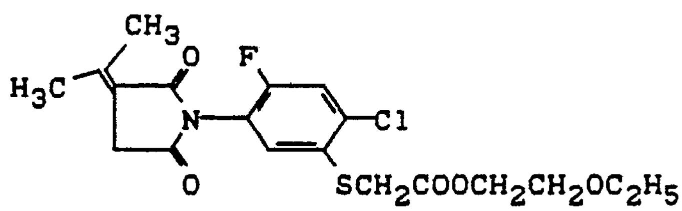

Verfahren (c)

Man suspendiert 2,86 g (0,008 Mol) 1-(4-Chlor-5-carboxymethylthio-2-fluorphenyl)-3-isopropylidenbernsteinsäureimid in 15 ml 2-Ethoxyethanol und leitet bei 5 °C trockenes HCl-Gas bis zur Sättigung ein. Man rührt eine Stunde bei Raumtemperatur nach und erhitzt anschließend noch eine Stunde bei 50 °C. Nach Einengen nimmt man den Rückstand in Methylenchlorid auf und wäscht mit einer 10 %igen Natriumhydrogencarbonatlösung. Man trocknet über Natriumsulfat und engt ein. Durch säulenchromatografische Reinigung über Kieselgel erhält man 2,44 g (71,0 % d. Th.) 1-[4-Chlor-5-(2-ethoxyethoxycarbonylmethylthio)-2-fluorphenyl]-3-isopropylidenbernsteinsäureimid als Öl.
$^1$H-NMR (80 Mhz, $CDCl_3$, δ): 7,43 (d, 1H, J = 8,0 Hz); 7,33 (d, 1H, J = 9,0 Hz); 4,26 (t, 2H, J = 5,0 Hz); 3,69 (s, 2H); 3,59 (t, 2H, J = 5,0 Hz); 3,49 (g, 2H, J = 8,0 Hz); 3,41 (bs, 2H); 2,38 (s, 3H); 1,95 (s, 3H); 1,18 (t, 3H, J = 8,0 Hz).

Beispiel 7

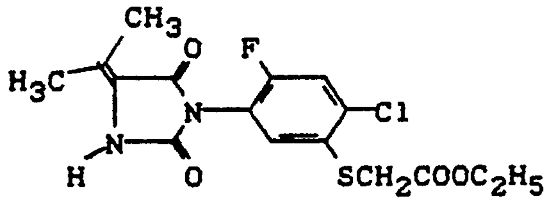

Verfahren (d)

Zu einem Gemisch aus 5,54 g (0,02 Mol) N-Benzyloxycarbonyl-α,β-dehydrovalinethylester, 2,02 g (0,02 Mol) Triethylamin und 20 ml o-Dichlorbenzol gibt man 5,79 g (0,02 Mol) 2-Fluor-4-chlor-5-ethoxycarbonylmethylthiophenylisocyanat hinzu und erhitzt anschließend das Reaktionsgemisch 15 Stunden bei 140 °C. Nach Abkühlen engt man ein und reinigt den Rückstand durch Säulenchromatografie über Kieselgel (Laufmittel Ethylacetat/Petrolether). Man erhält 1,54 g (19,9 % d. Th.) 3-(2-Fluor-4 chlor-5-ethoxycarbonylmethylthiophenyl)-5-isopropyliden-3H-imidazol-2,4-dion vom Schmelzpunkt 162 - 169 °C.
$^1$H-NMR (80 Mhz, $CDCl_3$, δ): 9,05 (s, 1H), 7,48 (d, 1H, J=8,0 Hz); 7,34 (d, 1H, J=9,0 Hz); 4,17 (g, 2H, J=8,0 Hz); 3,66 (s, 2H); 2,29 (s, 3H); 1,90 (s, 3H); 1,22 (t, 3H, J=8,0 Hz).

Beispiel 8

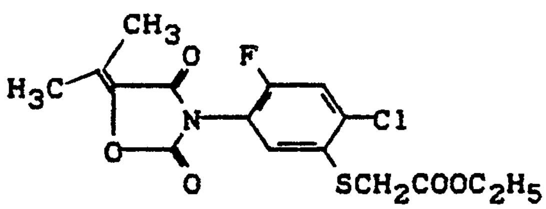

Verfahren (d)

Vorprodukt : Umsetzungsprodukt von Carbonsäureester der Formel (VI) mit Iso(thio)cyanat der Formel (VII):

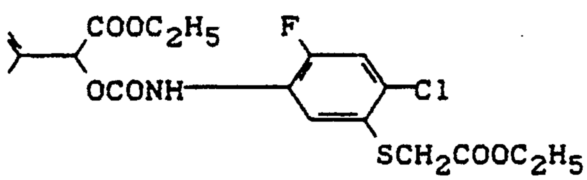

Eine Lösung von 3,60 g (0,025 Mol) 2-Hydroxy-3-methyl-3-butensäureethylester und 4,34 g (0,015 Mol) 2-Fluor-4-chlor-5-ethoxycarbonylmethylthiophenylisocyanat in 50 ml Toluol wird sechs Stunden bei 80 °C erhitzt. Nach Abkühlen engt man ein und trennt den Rückstand durch Säulenchromatografie über Kieselgel (Laufmittel Ethylacetat/Petrolether) auf. Man erhält 1,52 g (23,4 % d. Th.) 2-[N-(2-Fluor-4-chlor-5-ethoxycarbonylmethylthiophenyl)carbamoyloxy]-3-methyl-3-butensäureethylecter als Öl.
$^1$H-NMR (80 Mhz, $CDCl_3$, δ); 8,23 (d, 1H, J=8,2 Hz); 7,18 (bs, 1H); 7,16 (d, 1H, J=9,0 Hz); 5,47 (s, 1H); 5,24 (bs, 1H); 5,15 (bs, 1H); 4,25 (g, 2H, J=8,0 Hz); 4,19 (g, 2H, J=8,0 Hz); 3,66 (c, 2H); 1,84 (c, 3H); 1,30 (t, 3H, J=8,0Hz); 1,25 (t, 3H, J=8,0 Hz).

Umsetzung zu Beispiel 8

Man löst 0,87 g (0,002 Mol) des oben beschriebenen Vorproduktes in 12 ml Toluol, fügt 0,07 g (0,001 Mol) Natriumethylat hinzu und kocht noch fünf Stunden bei Rückflußtemperatur. Nach Abkühlen engt man ein.

Man erhält 0,65 g (86 % d. Th.) 3-(2-Fluor-4-chlor-5-ethoxycarbonylmethylthiophenyl)-5-isopropyliden-1,3-oxazolidin-2,4-dion.

$^1$H-NMR (80 Mhz, $CDCl_3$, $\delta$): 7,49 (d, 1H, J=8,0 Hz); 7,36 (d, 1H, J=9,0 Hz); 4,20 (g, 2H, J=8,0 Hz); 3,65 (s, 2H); 2,30 (s, 3H); 2,06 (s, 3H); 1,23 (t, 3H, J=8,0 HZ).

Beispiel 9

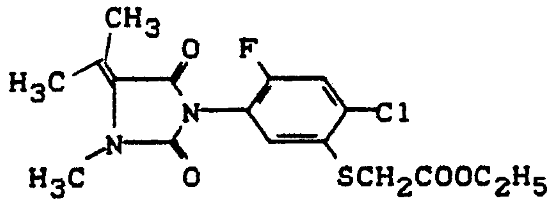

Verfahren (e)

Ein Gemisch aus 0,97 g (0,0025 Mol) 3-(2-Fluor-4-chlor-5-ethoxycarbonylmethylthiophenyl)-5-isopropyliden-1H-imidazol-2,4-dion, 0,52 g (0,00375 Mol) Kaliumcarbonat und 15 ml Acetonitril wird zwei Stunden auf Rückfluß erhitzt. Man kühlt ab und tropft bei 25° C 0,71 g (0,005 Mol) Methyliodid hinzu. Nach vier Stunden Rückfluß engt man ein und extrahiert den Rückstand mit Methylenchlorid. Man wäscht die organische Phase mit einer gesättigten Natriumhydrogencarbonatlösung und Wasser, trocknet über Natriumsulfat und engt ein. Nach Umkristallisieren aus einem Gemisch von Methylenchlorid/Petrolether erhält man 0,99 g (99,0 % d. Th.) 3-(2-Fluor-4-chlor-5-ethoxycarbonylmethylthiophenyl)-5-isopropyliden-1-methylimidazol-2,4-dion als Öl.

$^1$H-NMR (80 Mhz, $CDCl_3$, $\delta$): 7,46 (d, 1H, J=8,0 Hz); 7,32 (d, 1H, J=9,0 Hz); 4,17 (g, 2H, J=8,0 HZ); 3,64 (s, 2H); 3,45 (s, 3H); 2,36 (s, 3H); 2,20 (s, 3H); 1,22 (t, 3H, J=8,0 Hz).

- 62 -

Analog zu den Beispielen 1 bis 9 sowie nach der allgemeinen Beschreibung der erfindungsgemäßen Herstellungsverfahren können die in der nachstehenden Tabelle 2 aufgeführten Verbindungen der Formel (I) erhalten werden.

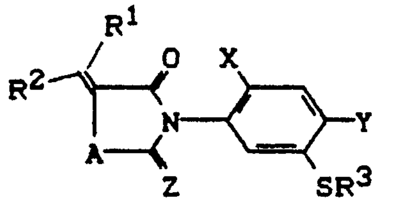

(I)

Tabelle 2

Herstellungsbeispiele für die Verbindungen der Formel (I)

| Bsp.-Nr. | $R^1$ | $R^2$ | $R^3$ | X | Y | Z | A | Schmelzpunkt °C |
|---|---|---|---|---|---|---|---|---|
| 10 | $CH_3$ | $CH_3$ | $CH_2COOH$ | H | Cl | O | $CH_2$ | 125-129 |
| 11 | $CH_3$ | $CH_3$ | $CH_2COOCH_3$ | H | Cl | O | $CH_2$ | 120-124 |
| 12 | $CH_3$ | $CH_3$ | $CH_2COOC_2H_5$ | H | Cl | O | $CH_2$ | 124-130 |
| 13 | $CH_3$ | $CH_3$ | $CH_2CN$ | H | Cl | O | $CH_2$ | 156-165 |
| 14 | $CH_3$ | $CH_3$ | $CHCOOC_2H_5$<br>$CH_3$ | H | Cl | O | $CH_2$ | Öl |
| 15 | $CH_3$ | $CH_3$ | $CH_2C{\equiv}CH$ | H | Cl | O | $CH_2$ | 180-18 |
| 16 | $CH_3$ | $CH_3$ | $CF_3$ | H | Cl | O | $CH_2$ | 100-102 |
| 17 | $CH_3$ | $CH_3$ | $CH_2COOCH_3$ | F | Cl | O | $CH_2$ | 83- 86 |
| 18 | $CH_3$ | $CH_3$ | $CH_2COOC_2H_5$ | F | Cl | O | $CH_2$ | 86- 90 |
| 19 | $CH_3$ | $CH_3$ | $CH_2COOCH(CH_3)_2$ | F | Cl | O | $CH_2$ | Öl |
| 20 | $CH_3$ | $CH_3$ | $CHCOOH$<br>$CH_3$ | F | Cl | O | $CH_2$ | Harz |
| 21 | $CH_3$ | $CH_3$ | $CHCOOC_2H_5$<br>$CH_3$ | F | Cl | O | $CH_2$ | Öl |
| 22 | $CH_3$ | $CH_3$ | $CH_2CH_2COOC_2H_5$ | F | Cl | O | $CH_2$ | Öl |
| 23 | $CH_3$ | $CH_3$ | $CH_2C{\equiv}CH$ | F | Cl | O | $CH_2$ | 150-15 |
| 24 | $CH_3$ | $CH_3$ | $CH_2COOC_2H_5$ | F | Br | O | $CH_2$ | 83- 87 |
| 25 | $CH_3$ | $CH_3$ | $CHCOOC_2H_5$<br>$CH_3$ | F | Br | O | $CH_2$ | Öl |

Anwendungsbeispiele

In den folgenden Anwendungsbeispielen wurde die nachstehend aufgeführte Verbindung (A) als Vergleichssubstanz herangezogen:

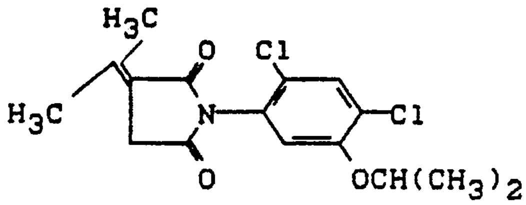

(A)

(bekannt aus EP-A 190 755).

Beispiel A

Pre-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstante. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber der bekannten Verbindung (A) zeigen in diesem Test z.B. die Verbindungen gemäß den Herstellungsbeispielen 6, 15, 17 und 23.

Beispiel B

Post-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit gegenüber der bekannten Verbindung N-(2,4-Dichlor-5-isopropoxyphenyl)-3-isopropylidenbernsteinsäureimid zeigen in diesem Test z.B. die Verbindungen gemäß den Herstellungsbeispielen 3, 6, 14, 17, 18, 19, 22 und 24.

Beispiel C:

Entlaubung und Austrocknung der Blätter bei Baumwolle
Lösemittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösemittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach einer Woche werden Blattfall und Austrocknen der Blätter im Vergleich zur Kontrolle bonitiert.

In diesem Test zeigen z.B. die Verbindungen gemäß den Herstellungsbeispielen 3, 6 und 18 eine gute Wirkung.

## Ansprüche

1. N-(5-Alkylthiophenyl)-Stickstoffheterocyclen der allgemeinen Formel (I)

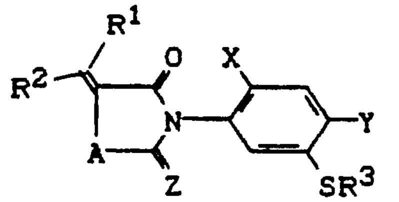

(I)

in welcher

$R^1$ und $R^2$ entweder unabhängig voneinander jeweils für Wasserstoff oder Alkyl stehen oder gemeinsam für einen zweifach verknüpften Alkandiylrest stehen,

$R^3$ für Wasserstoff, für jeweils gegebenenfalls substituiertes Alkyl, Alkenyl, Alkinyl, Cycloalkyl, Arylalkyl oder Heteroarylalkyl steht,

X für Wasserstoff oder Halogen steht,

Y für Halogen oder Alkyl steht,

Z für Sauerstoff oder Schwefel steht,

A für Sauerstoff, für eine CH-$R^4$-Gruppe, für eine

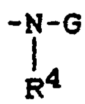-Gruppe oder für eine 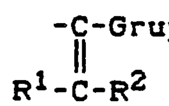-Gruppe steht,

wobei

$R^4$ für Wasserstoff, für jeweils gegebenenfalls substituiertes Alkyl, Alkenyl, Alkinyl, Cycloalkyl, Aryl oder Alkoxycarbonyl steht.

2. N-(5-Alkylthiophenyl)-Stickstoffheterocyclen der Formel (I) gemäß Anspruch 1, in welcher

$R^1$ und $R^2$ entweder unabhängig voneinander jeweils für Wasserstoff oder für geradkettiges oder verzweigtes Alkyl mit 1 bis 4 Kohlenstoffatomen stehen oder gemeinsam für einen zweifachverknüpften Alkandiylrest mit 2 bis 7 Kohlenstoffatomen stehen,

$R^3$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonyl alkyl, Alkylthiocarbonylalkyl, Dialkylaminocarbonylalkyl, Alkoxycarbonylalkyl, Alkoxycarbonylhalogenalkyl, Carboxyalkyl, Carboxyhalogenalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils in Frage kommen: Halogen sowie jeweils geradkettiges oder verzweigtes Alkyl oder

Alkoxy mit jeweils 1 bis 4 Kohlenstoffatomen, $R^3$ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit jeweils 1 bis 3 Kohlenstoffatomen in den jeweiligen Alkylteilen steht und schließlich für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten in Frage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
X für Wasserstoff, Fluor, Chlor oder Brom steht,
Y für Fluor, Chlor, Brom, Methyl oder Ethyl steht,
Z für Sauerstoff oder Schwefel steht,
A für Sauerstoff, für eine -CH-$R^4$-Gruppe, für

eine -N-Gruppe oder für eine -C-Gruppe steht,

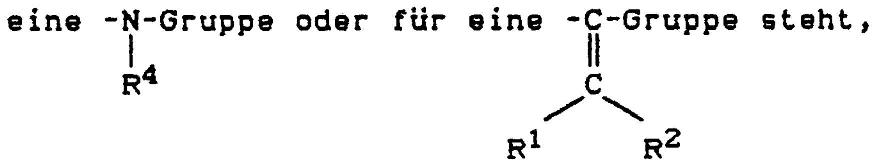

wobei
$R^4$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten im Cycloalkylteil jeweils in Frage kommen: Halogen sowie jeweils geradkettiges oder verzweigtes Alkyl oder Alkoxy mit jeweils 1 bis 4 Kohlenstoffatomen.

3. N-(5-Alkylthiophenyl)-Stickstoffheterocyclen der Formel (I) gemäß Anspruch 1, in welcher
$R^1$ und $R^2$ entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen,
$R^3$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl, Alkylthiocarbonylalkyl, Dialkylaminocarbonylalkyl, Carboxyalkyl, Carboxyhalogenalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht, oder für jeweils gegebenenfalls ein- bis dreifach, gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,
X für Wasserstoff, Fluor oder Chlor steht,
Y für Fluor, Chlor oder Brom steht,
Z für Sauerstoff oder Schwefel steht,

A für Sauerstoff, für eine -CH-$R^4$-Gruppe, für

eine -N-Gruppe oder für eine -C-Gruppe steht,

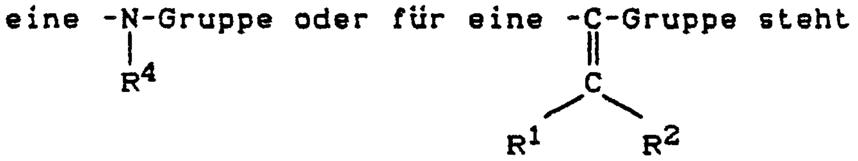

wobei

$R^4$ für Wasserstoff, für Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder ver zweigtes Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich und verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, oder

$R^4$ für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht.

4. N-(5-Alkylthiophenyl)-Stickstoffheterocyclen der Formel (I) gemäß Anspruch 1, in welcher

$R^1$ und $R^2$ entweder unabhängig voneinander jeweils für Wasserstoff oder Methyl oder gemeinsam für einen Ethan-1,2-diylrest, einen Butan-1,4-diylrest oder einen Pentan-1,5-diylrest stehen und

$R^3$ für Wasserstoff, Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen steht, für jeweils geradkettiges oder verzweigtes Cyanalkyl, Alkoxyalkyl, Alkoxyalkoxyalkyl, Carboxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetra hydrofuranylmethyl oder Tetrahydropyranylmethyl steht oder für jeweils gegebenenfalls ein- bis dreifach, gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

X für Wasserstoff oder Fluor steht,

Y für Chlor oder Brom steht,

Z für Sauerstoff oder Schwefel steht,

A für Sauerstoff, für eine -CH-$R^4$-Gruppe, für

eine -N-Gruppe oder für eine -C-Gruppe steht

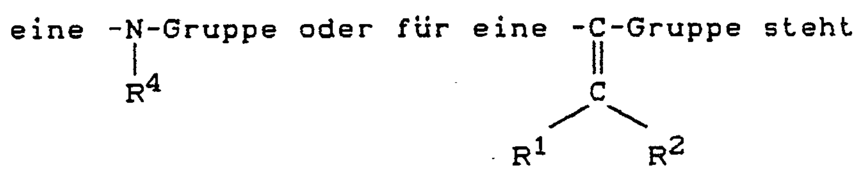

und

$R^4$ für Wasserstoff, Methyl, Ethyl, n- oder i-Propyl, n-, i-, s- oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen steht, für jeweils geradkettiges oder verzweigtes Cyanalkyl, Alkoxyalkyl, Alkoxyalkoxyalkyl oder

Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor und Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl oder Tetrahydropyranylmethyl steht.

5. Verfahren zur Herstellung von N-(5-Alkylthiophenyl)-Stickstoffheterocyclen der allgemeinen Formel (I)

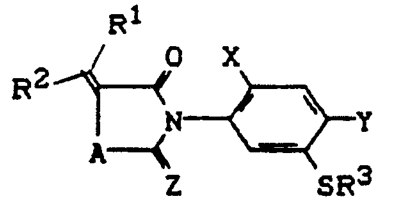

(I)

in welcher

$R^1$ und $R^2$ entweder unabhängig voneinander jeweils für Wasserstoff oder Alkyl stehen oder gemeinsam für einen zweifach verknüpften Alkandiylrest stehen,

$R^3$ für Wasserstoff, für jeweils gegebenenfalls substituiertes Alkyl, Alkenyl, Alkinyl, Cycloalkyl, Arylalkyl oder Heteroarylalkyl steht,

X für Wasserstoff oder Halogen steht,

Y für Halogen oder Alkyl steht,

Z für Sauerstoff oder Schwefel steht,

A für Sauerstoff, für eine CH-$R^4$-Gruppe, für eine

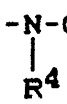-Gruppe oder für eine 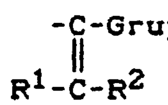-Gruppe steht,

wobei

$R^4$ für Wasserstoff, für jeweils gegebenenfalls substituiertes Alkyl, Alkenyl, Alkinyl, Cycloalkyl, Aryl oder Alkoxycarbonyl steht,

dadurch gekennzeichnet, daß man

a) N-(5-Alkylthiophenyl)-Stickstoffheterocyclen der Formel (Ia),

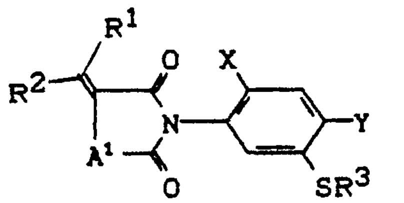

(Ia)

in welcher

$A^1$ für eine -CH-$R^4$-Gruppe oder für eine

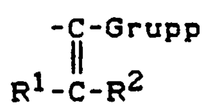-Gruppe

steht und

$R^1$, $R^2$, $R^3$, $R^4$, X und Y die oben angegebene Bedeutung haben,

erhält, wenn man Alkylidenbernsteinsäureanhydride der Formel (II),

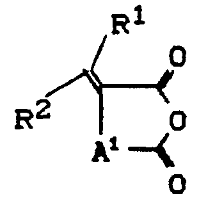

(II)

in welcher $A^1$, $R^1$ und $R^2$ die oben angegebene Bedeutung haben, mit Anilinen der Formel (III),

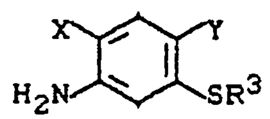

(III)

in welcher X, Y und $R^3$ die oben angegebene Bedeutung haben, gegebenenfalls in Gegenwart eines Verdünnungsmittels und gegebenenfalls in Gegenwart eines Reaktionshilfsmittels umsetzt; oder daß man

b) die nach Verfahren a) erhältlichen N-(5-Alkylthiophenyl)-Stickstoffheterocyclen der Formel (Ib)

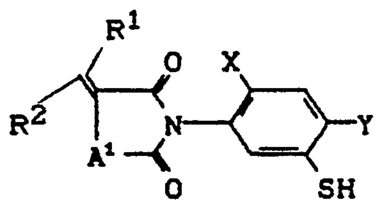

(Ib)

in welcher $R^1$, $R^2$, X, Y und $A^1$ die oben angegebene Bedeutung haben, mit Alkylierungsmitteln der Formel (IV)

$R^3$-$E^1$ (IV)

in welcher $E^1$ für eine elektronenanziehende Abgangsgruppe steht und $R^3$ die oben angegebene Bedeutung hat, gegebenenfalls in Gegenwart eines Verdünnungsmittels und gegebenenfalls in Gegenwart eines Reaktionshilfsmittels umsetzt; oder daß man

c) die nach Verfahren a) erhältlichen N-(5-Alkylthiophenyl)-Stickstoffheterocyclen der Formel (Ic),

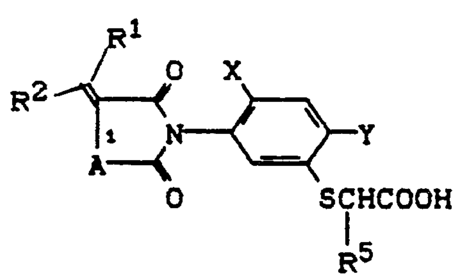

(Ic)

in welcher $R^1$, $R^2$, X, Y und $A^1$ die oben angegebene Bedeutung haben und $R^5$ für Wasserstoff, Halogen oder Alkyl steht, mit Alkoholen der Formel (V),

$R^6OH$ (V)

in welcher

$R^5$ für jeweils gegebenenfalls substituiertes Alkyl, Alkenyl, Alkinyl oder Cycloalkyl steht, gegebenenfalls in Gegenwart eines Verdünnungsmittels und gegebenenfalls in Anwesenheit einer Säure umsetzt; oder daß man

d) N-(5-Alkylthiophenyl)-Stickstoffheterocyclen der Formel (Id),

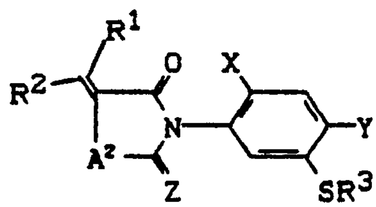 (Id)

in welcher

$R^1$, $R^2$, $R^3$, X, Y und Z die oben angegebene Bedeutung haben und

$A^2$ für eine

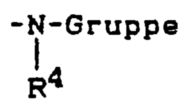

oder für Sauerstoff steht, wobei $R^4$ die oben angegebene Bedeutung hat, erhält, wenn man Carbonsäureester der Formel (VI),

$R^7$-$COOR^8$ (VI)

in welcher

$R^7$ für einen Rest der Formel

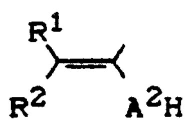 oder 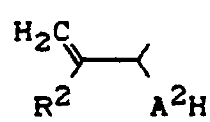 und

$R^8$ für Alkyl steht, wobei

$R^1$, $R^2$ und $A^2$ die oben angegebene Bedeutung haben,

oder deren Säureadditionssalze

mit Iso(thio)-cyanaten der Formel (VII)

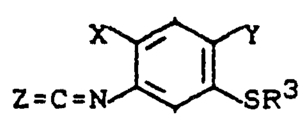 (VII)

in welcher

$R^3$, X, Y und Z die oben angegebene Bedeutung haben,

gegebenenfalls in Gegenwart eines Verdünnungsmittels und gegebenenfalls in Gegenwart eines Reaktionshilfsmittels umsetzt; oder daß man

e) die nach Verfahren (d) erhältlichen N-(5-Alkylthiophenyl)-Stickstoffheterocyclen der Formel (Ie)

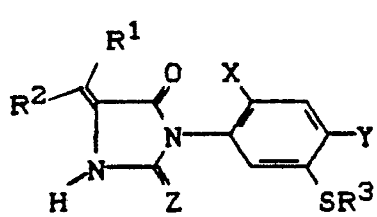 (Ie)

in welcher
$R^1$, $R^2$, $R^3$, X, Y und Z die oben angegebene Bedeutung haben,
mit Alkylierungsmitteln der Formel (VIII),
$R^{4-1}$-$E^2$ (VIII)
in welcher
$E^2$ für eine elektronenanziehende Abgangsgruppe steht und
$R^{4-1}$ für jeweils gegebenenfalls substituiertes Alkyl, Alkenyl, Alkinyl, Cycloalkyl oder Alkoxycarbonyl steht,
gegebenenfalls in Gegenwart eines Verdünnungsmittels und gegebenenfalls in Gegenwart eines Reaktionshilfsmittels umsetzt.

6. Herbizide und pflanzenwuchsregulierende Mittel, gekennzeichnet durch einen Gehalt an mindestens einem N-(5-Alkylthiophenyl)-Stickstoffheterocyclus der Formel (I) gemäß Anspruch 1 oder 5.

7. Verfahren zur Bekämpfung von unerwünschten Pflanzen und zur Regulierung des Pflanzenwuchses, dadurch gekennzeichnet, daß man N-(5-Alkylthiophenyl)- Stickstoffheterocyclen der Formel (I) gemäß Anspruch 1 oder 5 auf die Pflanzen und/oder ihren Lebensraum einwirken läßt.

8. Verwendung von N-(5-Alkylthiophenyl)-Stickstoffheterocyclen der Formel (I) gemäß Anspruch 1 oder 5 zur Bekämpfung von unerwünschten Pflanzen und/oder als Pflanzenwuchsregulatoren.

9. Verfahren zur Herstellung von herbiziden und/oder pflanzenwuchsregulierenden Mitteln, dadurch gekennzeichnet, daß man N-(5-Alkylthiophenyl)-Stickstoffheterocyclen der Formel (I) gemäß Anspruch 1 oder 5 mit Streckmitteln und/oder oberflächenaktiven Substanzen Vermischt.

Europäisches Patentamt

# EUROPÄISCHER RECHERCHENBERICHT

Nummer der Anmeldung

**EP 90 11 5966**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 190 755 (KASEI)<br>* Zusammenfassung; Ansprüche 1-19 *<br>– – – | 1-9 | C 07 D 207/452<br>C 07 D 207/02<br>C 07 D 233/96<br>C 07 D 263/44<br>A 01 N 43/36<br>A 01 N 43/76<br>A 01 N 43/50 |
| A | US-A-4 582 903 (S.B. MIRVISS)<br>* Insgesamt *<br>– – – | 1-9 | |
| A | US-A-3 853 912 (D.E. BUBLITZ)<br>* Insgesamt *<br>– – – | 1-9 | |
| A | EP-A-0 288 789 (BAYER)<br>* Insgesamt *<br>– – – | 1-9 | |
| A | DE-A-3 642 372 (DOW CHEMICAL)<br>* Insgesamt *<br>– – – | 1-9 | |
| A | EP-A-0 049 841 (UBE INDUSTRIES)<br>* Insgesamt *<br>– – – | 1-9 | |
| A | EP-A-0 262 428 (SAGAMI)<br>* Insgesamt *<br>– – – | 1-9 | RECHERCHIERTE SACHGEBIETE (Int. Cl.5) |
| A | EP-A-0 241 559 (SAGAMI)<br>* Insgesamt *<br>– – – – – | 1-9 | C 07 D 207/00<br>C 07 D 233/00<br>C 07 D 263/00 |

Der vorliegende Recherchenbericht wurde für alle Patentansprüche erstellt

| Recherchenort | Abschlußdatum der Recherche | Prüfer |
|---|---|---|
| Den Haag | 21 November 90 | KISSLER B.E. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument